# DEMANDE DE BREVET EUROPEEN

(11) **EP 0 699 433 A2**
(43) Date de publication de la demande: **06.03.1996**
(21) Numéro de dépôt: 95420177.8
(22) Date de dépôt: 30.06.1995
(51) Int. Cl.: A61K 7/48, A61K 31/73

(54) **Composition cosmétique ou pharmaceutique contenant des dérivés du chitosane**

(30) Priorité: 30.06.1994 FR 9408314
(71) Demandeur: GATTEFOSSE S.A., F-69800 Saint Priest (FR)
(72) Inventeur: Rinaudo, Marguerite, F-38000 Grenoble (FR); Desbrieres, Jacques, F-38500 Voiron (FR); Klein, Jean-Marie, F-69320 Feyzin (FR); Mahler, Bruno, F-69300 Caluire (FR)
(74) Mandataire: Laurent, Michel

(57) **Abrégé**

Composition cosmétique ou pharmaceutique contenant de 0,1 à 5 % en poids d'un polymère dérivé du chitosane, constitué de plusieurs motifs monomères répartis au hasard dans la molécule du polymère, respectivement :
A - un motif glucosamine N-acétylé ou N-alkylé,
B - un motif glucosamine
   caractérisée en ce que ledit polymère contient au moins deux autres motifs monomères, respectivement :
C - en quantité majoritaire, un motif glucosamine-O-carboxyméthylé ou glucosamine N-acétylé O-carboxyméthylé
et D - un motif glucosamine-O-carboxyméthylé N-alkylé.

## Description

### Domaine Technique

L'invention concerne un nouveau type de composition cosmétique ou pharmaceutique contenant des dérivés du chitosane, c'est-à-dire de la chitine désacétylée soluble en milieu acide, présentant un degré d'acéthylation (DA) au plus égal à 40 %.

### Techniques antérieures

Comme on le sait, la chitine ou poly-(N-acétyle-D-glucosamine) est le polysaccharide le plus abondant sur la terre avec la cellulose. Ce polymère, bien que largement répandu, présente l'inconvénient d'être insoluble dans l'eau.

Le chitosane est le produit de la désacétylation de la chitine dont les groupes N-acétylés en C₂ sont désacétylés par des réactifs alcalins. Du fait de la présence des groupes amines libres, le chitosane, polymère cationique (en milieu acide), est soluble dans l'eau à pH acide. Dans le but d'améliorer la solubilité du chitosane en milieu alcalin, on a proposé de modifier sa molécule (voir par exemple document FR-A-2 137 684).

Dans le document EP-A-0 371 878, on a décrit des dérivés du chitosane O-carboxylés organosolubles constitués par un enchaînement statistique de deux motifs spécifiques. Ces dérivés, utiles comme agents séquestrants, pour la préparation de films ou pour l'enrobage d'additifs alimentaires pour ruminants, sont solubles dans les solvants organiques usuels. En outre, ces polymères dérivés du chitosane ont un faible poids moléculaire (inférieur à 80 000, en pratique compris entre 10 et 20 000), ce qui nuit à la stabilité des émulsions réalisées à partir de ces polymères, stabilité exigée dans le domaine de la cosmétique.

Dans le document US-A-4 027 068, on a proposé d'utiliser des dérivés du chitosane O-carboxyalkylés ou N-carboxylés, comme agents anti-buée pour les pare-brise automobiles. Ces dérivés sont obtenus par O-carboxylation, puis N-alkoxylation au moyen de composés alkyl halogénés. Ce type de réaction ne permet pas de substituer efficacement des radicaux alkyls à longue chaine (en C₁₂ et plus), nécessaires pour obtenir une balance hydrophile-hydrophobe appropriée pour des applications cosmétiques. Les polymères obtenus sont détergents, transparents, solubles dans l'eau, mais hygroscopiques, ce qui ne permet pas de les utiliser efficacement dans des applications cosmétiques. En outre, produits cosmétiques et agents anti-buée de pare-brise appartiennent à des domaines techniques différents et fort éloignés.

L'invention vise à obtenir des polymères dérivés du chitosane, constitués de plusieurs motifs monomères différents, répartis au hasard dans la molécule du polymère du chitosane, qui présentent un pouvoir émulsionnant, des propriétés gélifiantes améliorées et une très grande onctuosité, propriété de plus en plus recherchée dans le domaine de la cosmétologie.

### Description sommaire de l'Invention

L'invention concerne une composition cosmétique ou pharmaceutique contenant de 0,1 à 5 % en poids d'un polymère dérivé du chitosane constitué de plusieurs motifs monomères répartis au hasard dans la molécule du polymère, respectivement :
A - un motif glucosamine N-acétylé ou N-alkylé 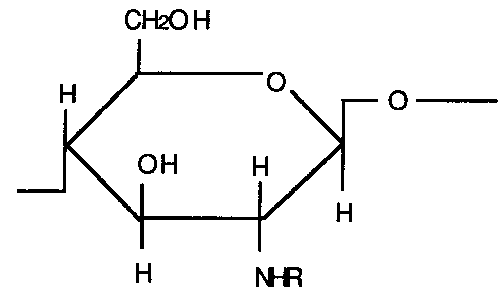 avec R égal à -COCH₃ ou (CH₂)n - CH₃
B - un motif glucosamine 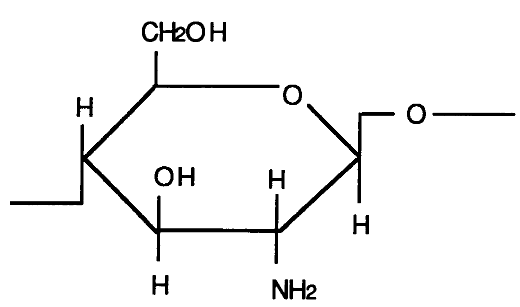 caractérisée en ce que ledit polymère contient au moins deux autres motifs monomères, respectivement :
C - en quantité majoritaire, un motif glucosamine-O-carboxyméthylé ou glucosamine N-acétylé O-carboxyméthylé de formule générale : 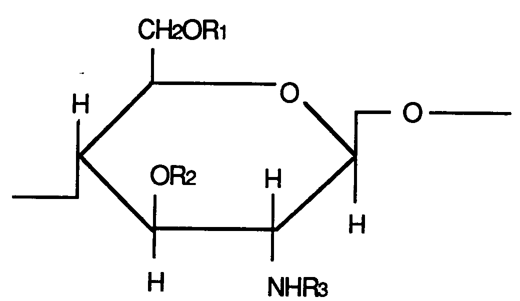
et D - un motif glucosamine-O-carboxyméthylé N-alkylé de formule générale : 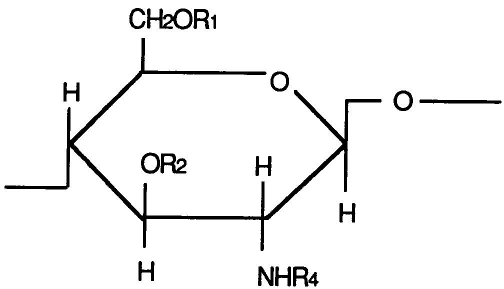 dans lesquels :
   . R₁ et R₂ désignent l'atome d'hydrogène ou le radical -CH₂COOX, dans lequel X désigne H ou un cation monovalent ;
   . et R₃ désigne H, un radical -COCH₃ ou le radical -CH₂COOX dans lequel X désigne H ou un cation monovalent;
   . et R₄ désigne un radical alkyl en C₂ à C₁₈, linéaire ou branché, saturé ou non.

En d'autres termes, l'invention vise un polymère dérivé du chitosane constitué d'au moins quatre motifs monomères répartis au hasard dans la molécule du polymère, à savoir deux motifs A et B glucosamine, déjà connus pour cette application, combinés avec deux autres motifs C, majoritaire, et D glucosamine-O-carboxyméthylé N-alkylé.

En d'autres termes, l'invention vise des compositions contenant des polymères dérivés du chitosane, dans lesquels les fonctions alcool primaire ou secondaire associées aux atomes de carbone no. 6 ou no. 3 du motif répétitif du chitosane subissent, majoritairement, une réaction d'O-carboxyméthylation, et dans lesquels le composé ainsi obtenu est greffé par un radical alkyle sur l'azote de la fonction amine primaire du motif glucosamine carboxyméthylé ou non.

Comme on le sait, l'O-carboxyméthylation du chitosane est une opération connue qui consiste à fixer un radical carboxyméthyl (-CH₂COOX) sur l'oxygène de la fonction alcool associée à l'atome de carbone 6 et/ou 3 du monomère du chitosane. Cette opération est décrite dans "Advances in chitin and chitosan, Ed Brine, Sanford and Zitakis, Elsevier (1992), 516-525". Cette opération de O-carboxymétylation apporte l'hydrophilie à la molécule, donc favorise la solubilisation en milieu alcalin sous forme de sel sodique par exemple.

La N-alkylation, est comme on le sait, une opération qui consiste à greffer une chaine alkyle sur l'azote de la fonction amine du motif répétitif du chitosane. De tels composés et le procédé de fabrication sont décrits par exemple dans les documents US-A-4 424 346 et US-A-4 027 068 cité dans le préambule.

On sait que le chitosane O-carboxyméthylé est soluble dans l'eau à pH neutre ou alcalin (pH > *7*) ou à pH très acide (pH < 2,5). On sait aussi (voir US-A-4 424 346) que le chitosane partiellement alkylé donne des solutions gélifiées dans l'acide acétique dilué.

On a constaté que, si l'on combine les opérations d'O-carboxyméthylation et de N-alkylation partielle du chitosane, le dérivé obtenu a des propriétés inattendues, telles que notamment :
- l'autoassociativité : les chaines hydrophobes greffées sur la chaine macromoléculaire peuvent s'associer lors de la mise en solution (dans l'eau par exemple) en mettant en jeu un phénomène thermodynamique appelé "interaction hydrophobe" ; ce phénomène est mis en évidence par une augmentation notable de la viscosité de la solution dans l'eau, lorsque la concentration en polymère devient supérieure à 0,3 à 0,4 % en poids ; pour ces concentrations, la viscosité augmente avec la température ;
- l'auto-associativité avec des agents tensio-actifs : les chaines hydrophobes des polymères s'associent à la partie hydrophobe des molécules tensio-actives et lorsque la concentration de celles-ci est suffisante, cela génère une augmentation de la viscosité, voire la prise en gel de la solution ;
- une meilleure solubilité, puisque le composé obtenu est soluble dans l'eau pure, les solutions d'acide dilué et dans les mélanges eau/solvant organique tels que eau/DMSO, eau/dioxane.. ;
- des propriétés émulsifiantes; en effet, lorsqu'une solution de ces polymères est ajoutée sous forte agitation à de l'huile de vaseline ou de l'isostéarate d'isostéaryle par exemple, une émulsion se forme et reste stable durant deux mois au moins à température ambiante ; la concentration de polymère utilisée pour cette application peut être aussi faible que 0,5 % en poids par rapport à l'émulsion finale ; cette stabilité n'est pas affectée par une augmentation de température jusqu'à au moins 50°C.

Ces propriétés dépendent du degré de O-carboxyméthylation (D.S.), c'est-à-dire du nombre moyen de motifs -CH₂COOX par motif moyen statistiquement C et D. Ce degré peut varier de 0,1 à 2, de la longueur de la chaine carbonée du groupement alkyle que l'on peut choisir entre C₂ et C₁₈ et du nombre de ces chaines réparties le long de la macromolécule ; de la qualité du chitosane que l'on définit par son degré d'acétylation résiduelle D.A. et la viscosité de ses solutions à 1 % dans l'acide acétique à 1 % ; D.A. est inférieur à 20 % et la viscosité est comprise entre 5 et 1500 m.Pa.s.

Le radical alkyl linéaire R₄ en C₂ à C₁₈, est de préférence C₆ à C1₂. En effet, on a constaté que avec des radicaux alkyl présentant moins de six atomes de carbone, les propriétés de chitosane étaient peu améliorées. Les aldéhydes de faible poids moléculaire (jusqu'à C₁₀) sont fortement odorantes.

C'est pourquoi, en pratique, on utilise avantageusement l'aldéhyde laurique (la lauraldéhyde).

Dans une forme de réalisation avantageuse :
- R₁ et R₂ est -CH₂COOX à un pourcentage variant de 10 à 100 % (DS compris entre 0,2 et 2), et de préférence entre 25 et 65 % (DS compris entre 0,5 et 1,3);
- R₃ est le radical acétyle avec un pourcentage compris entre 0 et 20, de préférence 5 et 10 % ;
- R₃ est H à au moins 50 % ;
- R₄ est un radical alkyle à un pourcentage inférieur à 50 %, et de préférence compris entre 6 et 8 % lorsque ce radical est en C₁₂.

Avantageusement :
- R₁ et R₂ sont -CH₂COONa à raison de 25 à 65 % ;
- R₃ est un radical acétyle pour un maximum de 10 % ;
- R₄ est le radical lauryle pour un pourcentage compris entre 6 et 8 %.

Dans une autre forme d'exécution avantageuse, R₁ et/ou R₂ et/ou R₃ est -CH₂COOX pour 50 % des monomères du polymère obtenu.

La quantité des monomères minoritaires A, B et D varie en fonction des propriétés souhaitées, notamment des degrés de substitution, donc de solubilisation dans l'eau.

Un procédé pour la préparation de ces composés consiste :
- dans un premier temps, à O-carboxyméthyler le chitosane ;
- puis, à N-alkyler le composé obtenu.

Dans une variante, on peut tout d'abord N-alkyler le chitosane, puis O-carboxyméthyler le composé obtenu.

Les polymères obtenus de la sorte conformes à l'invention sont avantageux. Ils se présentent sous forme d'une poudre de couleur généralement crème, de poids moléculaire compris entre 20 et 800 000, de préférence voisin de 200 000, solubles dans l'eau à température ambiante à pH inférieur à 6 ou supérieur à 8,5, notamment sous agitation. Ces valeurs de pH dépendent du pourcentage d'alkylation et du degré de substitution (DS) de la réaction de carboxyméthylation.

Ces polymères se dissolvent dans l'eau à une concentration de 0,5 à deux pourcent (0,5 à 2 %) en poids pour conduire à des compositions ayant la consistance du miel.

Ces polymères présentent de nombreux avantages par rapport à ceux connus à ce jour. On peut citer :
- la possibilité, après incorporation dans une base de produits cosmétiques ou dans une émulsion, de conférer à celle-ci une stabilité recherchée, même à température élevée ;
- la formation de gels et d'émulsions.

De la sorte, ces composés peuvent être utilisés avec succès dans la fabrication de produits cosmétiques, voire pharmaceutiques.

La manière dont l'invention peut être réalisée et les avantages qui en découlent, ressortiront mieux des exemples de réalisation qui suivent.

### Manière de réaliser l'Invention

### Procédure générale

Le chitosane est préalablement purifié par dissolution dans de l'acide acétique dilué, filtration, précipitation à pH8 par neutralisation à l'aide de soude diluée et lavage du précipité par des solutions alcooliques de concentrations croissantes 70-80-95 % en volume.

### Réaction d'O-carboxyméthylation

Cette méthode est décrite dans la référence M Rinaudo, P Le Dung, M. Milas "A new and simple method of synthesis of carboxyméthylchitosan", 5th conference on Chitin and Chitosan, Ed-Brine, Sandford and Zitakir, Elsevier (1992) 516-525.

Un gramme (1 g) de chitosane est dissout dans 40 ml d'une solution aqueuse d'acide acétique à 1 %.

On additionne 40,3 g de solution aqueuse de NaOH à 50 % en poids sous forte agitation et une heure après 9 g de monochloracétate de Na .

La réaction dure de deux à quatre heures (2 à 4 h) à une température comprise entre 20 et 50°C sous agitation.

Le carboxyméthylchitosane dissout est précipité par l'éthanol (300 ml), centrifugé et lavé à l'alcool 70-80-95 % en volume, puis séché à température ambiante. Un séchage trop important nuit à la solubilité ultérieure du composé obtenu.

On mesure le degré de substitution (D.S.) obtenu par dosage potentiométrique et RMN. On obtient 0,1 à 1,30 selon les conditions utilisées.

### Réaction de N-alkylation

Le O-carboxyméthylchitosane sous forme de sel de Na⁺ (0,5 g, 2.10⁻³ monomole) est dissout dans un mélange d'eau distillée (40 ml) et d'éthanol (15 ml). L'aldéhyde en quantité voulue (x mole % par mole de chitosane) est ajoutée. La réaction a lieu (20h, 25°C) en présence de cyanoborohydrure de Na (0,4 g, 6.10⁻³ monomole).

Le composé recherché est précipité par l'éthanol (300 ml) purifié par deux étapes de précipitation, lavages successifs, puis séché à température ambiante.

Un séchage trop important nuit à la solubilité ultérieure du composé obtenu.

### Exemple 1 :

On utilise du chitosane de degré d'acétylation résiduel égal à 10 %.

Sa viscosité à 1 % en poids dans une solution d'acide acétique à 1 % en poids, est de 500 mPa.s.

La réaction a lieu à température ambiante entre 20 et 25°C pendant quatre heures.

On obtient un chitosane O-carboxyméthylé de DS = 0,5 soluble dans l'eau distillée. Celà signifie qu'en moyenne R₁ et/ou R₂ et/ou R₃ est -CH₂COOX pour 50 % des monomères du polymère obtenu.

La réaction d'alkylation est faite avec un aldéhyde en C₁₂ en proportion molaire par rapport à l'O-carboxyméthylchitosane de 0,08 ou 8%

On obtient un composé se présentant sous forme d'une poudre beige soluble dans l'eau à pH 8,5 sous forte agitation, pour donner une solution aqueuse qui gélifie à 2 %.

Ce composé répond statistiquement à la formule générale : 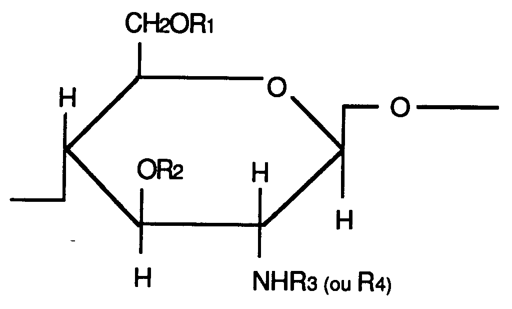
dans laquelle :
. R₁ et R₂ = H ou -CH₂COOX
. X = H ou Na selon pH < 5,5 ou > 8,5
. R₁ + R₂ = 25 % des motifs OH
. R₃ = H ou -COCH₃
. R₄ = C₁₂ dans 8 % des motifs répétitifs.

### Propriétés

Acidifiée à pH 5,5 avec l'acide lactique, une solution à 2 % se transforme en un gel translucide dont la viscosité mesurée au Rhéomat 115 est trois fois plus importante à 44°C qu'à 4°C, et présente un hystérésis, lorsqu'on mesure la contrainte en fonction du gradient de cisaillement en montée puis en descente, symbole d'une structure associée.

Une solution à 1 % ajustée à pH 5,5 par l'acide lactique est utilisée pour réaliser des émulsions à 5 % et 20 % d'huile de vaseline ou de trioctanoate de glycérol par agitation vigoureuse. Ces émulsions blanches ont l'aspect d'un gel sur lequel on n'observe pas de coalescence après deux semaines à température ambiante ou à 50°C. Elles ont un toucher particulièrement agréable propre à améliorer les émulsions cosmétiques (crèmes ou lait) avec les formulations ci-dessous :

| Lait épais | |
|---|---|
| agent émulsifiant à base d'alcools gras éthoxylés, commercialisé par le Demandeur sous la dénomination "Tefose 2000" | 7 % |
| Alcool cetylique | 1 % |
| huile de vaseline | 5 % |
| polymère dérivé de l'exemple 1 | 0,8 % |
| acide lactique | quantité suffisante pour obtenir un pH de 5,5 |
| Eau distillée | quantité suffisante pour ajuster à 100 % (QSP) |

Le polymère de l'exemple 1 est solubilisé dans l'eau distillée acidifiée à pH 5,5 par de l'acide lactique (par exemple), puis réchauffé à 70°C. L'ensemble des composés huileux chauffés à 70°C est additionné à la phase aqueuse sous agitation. L'émulsion est maintenue sous agitation pendant le refroidissement.

On obtient un lait épais, blanc brillant, onctueux au toucher, stable à 50°C pendant au moins cinq jours.

En l'absence du composé no. 1, le toucher est moins agréable d'une part, et cette formule est instable après vingt-quatre heures à température ambiante d'autre part, ce qui démontre bien les effets du composé no. 1.

| Lait fluide | |
|---|---|
| Isostéarate de polyéthylène glycol de poids moléculaire 300 | 5 % |
| huile d'amande douce | 15 % |
| polymère dérivé de l'exemple 1 | 0,4 % |
| acide lactique | QSP pH 5,5 |
| eau distillée | QSP 100 |

Le polymère de l'exemple 1 est solubilisé dans l'eau distillée, acidifiée à pH 5,5. La phase huileuse est incorporée à la phase aqueuse sous agitation à température ambiante. On obtient un lait fluide, brillant, stable à 50°C pendant au moins cinq jours.

En l'absence du composé no. 1, cette formule est instable à température ambiante et à chaud après vingt-quatre heures, ce qui montre bien l'effet du polymère dérivé de l'exemple 1 conforme à l'invention.

| Microémulsion (selon procédé décrit dans le document du Demandeur EP-B-0334 777) | |
|---|---|
| isostéarate de polyglycérol | 13,2 % |
| émulsifiant à base d'ester de polyéthylène glycol | 32,9 |
| isostéarate d'isostéaryl | 12,0 |
| eau | 21,8 |
| polymère dérivé de l'exemple 1 | 0,1 |
| eau | 20,0 |

Le polymère dérivé de l'exemple 1 est solubilisé dans la deuxème fraction d'eau distillée pour faire une solution à 0,5 %. Cette solution est incorporée au mélange des constituants précédents sous agitation, pour former une microémulsion translucide.

Une deuxième microémulsion est faite avec les mêmes constituants sans contenir toutefois le polymère dérivé caractéristique de l'invention.

La comparaison des microémulsions obtenues en application cutanée, fait ressortir une sensation de glissement améliorée en présence du polymère dérivé de l'exemple 1.

### Exemple 2 :

On utilise les mêmes matières premières et mêmes conditions que dans l'exemple 1, sauf la proportion d'aldéhyde C₁₂ qui devient 0,06 molaire.

Le composé no. 2 obtenu est une poudre beige soluble dans l'eau à pH 8,5, sous forte agitation, qui gélifie à 2 %. Acidifié à pH 5,5 par l'acide lactique une solution à 2 % devient un gel translucide.

Une solution à 1 % à pH 5,5, permet de réaliser des émulsions gélifiées avec 5 et 20 % d'huile de vaseline ou de trioctanoate de glycerol. Après une semaine à température ambiante ou à 50°C, on n'observe pas de coalescence.

### Exemples 3 à 8 :

Les résultats de tous les essais 1 à 8 sont rassemblés dans le tableau ci-annexé.

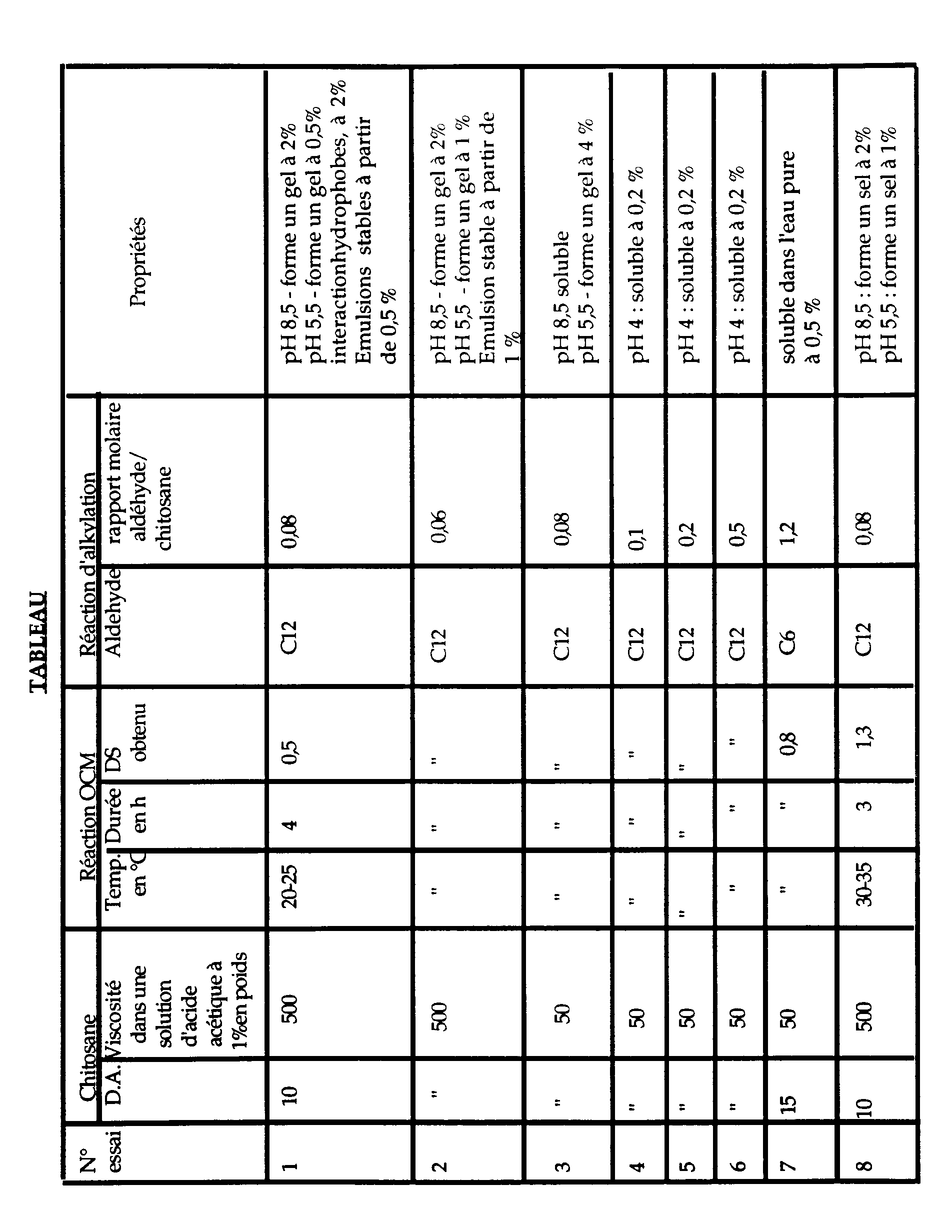

De ces exemples, on peut déduire que la combinaison des réactions d'O-carboxyméthylation et N-alkylation appliquées au chitosane de degré d'acétylation inférieur à 20 % et de viscosité supérieure ou égale à 50 mPa.s (en solution à 1 %), confère à ce polymère des propriétés particulièrement avantageuses.

Les composés selon l'invention sont solubles en milieu alcalin (pH > 8,5) ou acide (pH ≦ 5,5), même en solution diluée (0,5 %).

Les solutions plus concentrées se gélifient d'autant mieux que la température est plus élevée, en particulier à pH 5,5 ou inférieur.

On peut obtenir des émulsions stables pendant plusieurs jours, simplement en émulsifiant une huile dans une solution à 0,5 % ou plus de composé selon l'invention, pour obtenir une émulsion gélifiée, agréable à l'étalement cutané.

On peut également incorporer le composé selon l'invention dans des émulsions à usage cosmétique ou pharmaceutiques. A des concentrations de 0,5 % et 1 %, on obtient une amélioration considérable du toucher et de la consistance à température élevée (50°C).

On peut également incorporer le composé selon l'invention dans des microémulsions à usage cosmétique ou pharmaceutique.

A la concentration de 0,5 %, on obtient une amélioration de la sensation de glissement à l'application.

## Revendications

**1/** Composition cosmétique ou pharmaceutique contenant de 0,1 à 5 % en poids d'un polymère dérivé du chitosane, constitué de plusieurs motifs monomères répartis au hasard dans la molécule du polymère, respectivement :
A - un motif glucosamine N-acétylé ou N-alkylé 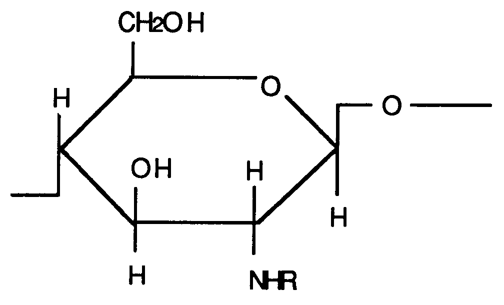 avec R égal à -COCH₃ ou (CH₂)ₙ - CH₃,
B - un motif glucosamine 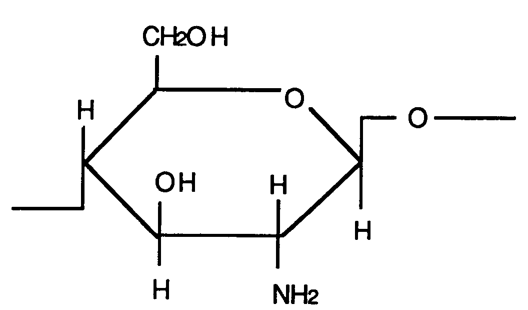 caractérisée en ce que ledit polymère contient au moins deux autres motifs monomères, respectivement :
C - en quantité majoritaire, un motif glucosamine-O-carboxyméthylé ou glucosamine N-acétylé O-carboxyméthylé de formule générale : 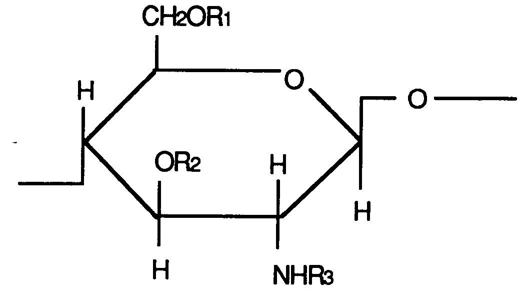
et D - un motif glucosamine-O-carboxyméthylé N-alkylé de formule générale 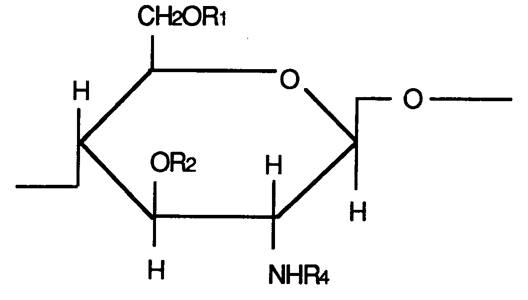 dans laquelle :
. R₁ et R₂ désignent l'atome d'hydrogène ou le radical -CH₂COOX, dans lequel X désigne H ou un cation monovalent ;
. R₃ désigne H, un radical -COCH₃ ou le radical -CH2COOX dans lequel X désigne H ou un cation monovalent ;
. et R₄ désigne un radical alkyl linéaire ou branché, saturé ou non, en C₂ à C₁₈.

**2/** Composition cosmétique ou pharmaceutique, selon la revendication 1, caractérisée en ce que dans le polymère dérivé du chitosane :
. R₁ est -CH₂COONa
. R₂ est H
. R₃ est H
. R₄ est le radical laurique.

**3/** Composition cosmétique selon la revendication 1, caractérisée en ce que dans le polymère dérivé du chitosane :
- R₁ et R₂ est -CH₂COOX pour un pourcentage variant entre 10 et 100 %, avec X = H ou un cation monovalent ;
- R₃ est au minimum de 50 % lorsque R3 est H et au maximum 20 % lorsque R₃ est un groupement acétyle ;
- R₄ est un radical alkyle à un pourcentage inférieur à 50 %, et de préférence compris entre 6 et 8 % lorsque R₄ est le radical lauryle.

**4/** Composition cosmétique selon l'une des revendications 2 et 3, caractérisée en ce que :
- R₁ et R₂ sont -CH₂COONa à raison de 25 à 65 % ;
- R₃ est un radical acétyle pour un maximum de 10 % ;
- R₄ est le radical lauryle pour un pourcentage compris entre 6 et 8 %.

**5/** Composition cosmétique selon la revendication 3, caractérisée en ce que R₁ et/ou R₂ et/ou R₃ est -CH₂COOX pour 50 % des monomères du polymère obtenu.
